Europäisches Patentamt

European Patent Office

Office européen des brevets

(11) Publication number: **0 252 191 B1**

## EUROPEAN PATENT SPECIFICATION

(45) Date of publication of patent specification: **17.06.92**   (51) Int. Cl.<sup>5</sup>: **A01G 9/10**

(21) Application number: **86305306.2**

(22) Date of filing: **10.07.86**

(54) Synthetic substrate for use in the rooting of cuttings and the raising of seedlings and plants.

(43) Date of publication of application:
**13.01.88 Bulletin 88/02**

(45) Publication of the grant of the patent:
**17.06.92 Bulletin 92/25**

(84) Designated Contracting States:
**AT BE CH DE FR IT LI LU NL SE**

(56) References cited:
**FR-A- 2 115 783**
**US-A- 4 215 513**

(73) Proprietor: **SYNTHETIC SUBSTRATES LIMITED**
**18 Ashley Road**
**Altrincham Greater Manchester(GB)**

(72) Inventor: **Perrin, Alan Philip**
**Cartref Gwenyn Cefy-y-Gwrych Meliden**
**Prestatyn Clwyd Wales(GB)**

(74) Representative: **Huntingford, David Ian et al**
**Geoffrey Owen & Company 76 Lower Bridge**
**Street**
**Chester CH1 1RU(GB)**

EP 0 252 191 B1

# Description

The present invention is concerned with synthetic substrates for use in the rooting of cuttings and the raising of seedlings and plants.

The traditional method of rooting cuttings and raising seedlings has involved the use of natural materials such as soil, peat and the like. In recent years, however, such natural materials have been replaced to a large extent by the use of synthetic plastics-based materials. These can be in a wide variety of forms, for example granular, shredded or cellular. It is with the latter, cellular type of material that the present invention is particularly concerned.

To provide the optimum conditions for either the successful rooting of cuttings or seed raising, a synthetic substrate must be permeable both to air and to the developing root structure. It must also be easily wettable by water and exhibit adequate levels of moisture retention.

Where the substrate is a continuous phase (as opposed to granular or shredded particles) cellular plastics material, for example phenolics or polyurethanes, permeability to air and the provision of adequate moisture (and moisture retention) are, to a significant extent, mutually exclusive objectives. This arises as a result of the poor capillarity of conventional cellular materials, consisting of substantially similar pore size.

For example, the manufacturers of expanded phenolic substrates recommend that the water "table" for a cellular block 50mm in height be maintained at 25mm., i.e. 50% of the height of the block. This is not only wasteful of water and energy (where bottom heat is provided due to latent heat effect) but also prevents air reaching the roots. This condition is found in practice to promote the undesirable proliferation of "watering" rather than "feeding" roots and increases the susceptibility of the cuttings or seedlings to root-rot.

It is a principal object of the present invention to provide an improved substrate for use in the rooting of cuttings and in the raising of seedlings, wherein the disadvantages of the known substrates are reduced significantly.

The present invention is based on the appreciation that the degree of capillarity of a hetero-cellular plastics material is related to its density - the higher the density, the higher the capillarity. It has been realised that this can be used to advantage to provide efficient watering and aeration of a cutting or seedling using a minimum of water, whilst reducing the likelihood of waterlogging.

In accordance with of the present invention, there is provided a synthetic substrate for use in the rooting of cuttings and the raising of seedlings, comprising an expanded, semi-rigid, substantially hetero-cellular plastics material having at least one region of relatively low density which exhibits relatively low capillarity and which is arranged, in use, to receive a cutting or seedling to be propagated and at least one region of relatively high density which exhibits higher capillarity than said low density region.

In one embodiment, the synthetic substrate comprises a plurality of said regions of relatively low density which are adapted to receive, in use, cuttings or seedlings to be propagated, the regions of low density being separated by the regions of relatively high density which exhibit higher capillarity than said regions of low density.

In one preferred embodiment, the substrate is in the form of a bandolier having a plurality of said low density regions, separated in the longitudinal direction of the bandolier by narrow strip regions of said higher density.

The low density regions may, for example, be of generally oval transverse section and may contain one or more blind bores for receiving cuttings or seedlings.

The synthetic substrate can be used as capillary matting for transmitting water and/or nutrients to plants, when constructed to have at least one said region of relatively low density which exhibits relatively low capillarity and on which, in use, one or more plant containers or trays can be placed, and at least one said region of relatively high density disposed below said region of relatively low density and exhibiting higher capillarity than said low density region.

Preferably, the cellular material is a polyurethane foam (either a polyester or a polyether) and the description which follows refers to the base material as being such. However, in practice it is equally possible to use some other foams having similar properties, such as expanded phenolics.

Polyurethanes are thermosetting materials, so that they cannot normally be subsequently thermoformed. If, however, a selected polyurethane is expanded to the degree implicit in a relatively low end density (for example 7.5 kgs/m$^3$), the thickness of the cell walls is reduced to the point where the compression set of the foam is very high. Therefore, when heat and pressure are applied to produce a desired profile, the subsequent deformation is, for all practical purposes, permanent (subject to a degree of dilation when wet).

Because the material is a thermoset, the deformed areas remain cellular. The size and therefore the number of cells per unit volume is, of course, a function of the degree of densification resulting from the induced permanent set.

Thus, in accordance with a further aspect of the invention, there is provided a method forming a synthetic substrate as defined above, wherein one or more predetermined regions of a block of foamed polyurethane or other suitable plastics of relatively low density are subjected to heat and pressure whereby to selectively compress and permanently deform the low density foamed material to produce said region or regions of relatively high density.

Differential densification results in differential pore (cell) size, which increases capillarity. The degree of capillarity can be enhanced further by the design of the profile resulting from said selective compression and deformation. Thus, narrow strips of increased capillarity between the regions of lower density containing the cuttings or seedlings can result in optimum water supply and retention without risk of waterlogging of the cuttings or seedlings themselves. By this means, the areas of greatest moisture retention potentiality can be varied to provide a desired moisture gradient, whilst preserving the required permeability to air.

Other advantages flow from this ability to differentially densify the polyurethane foam. The densified areas have good moisture capacity and retention since the rate of transpiration is decreased. These areas assist in reducing the danger of overwatering by "shedding" excess water. This latter feature is of particular signifcance where cutting are misted. In addition, the increased density, particularly in areas which have been reduced to minimum cross-section, exhibit considerably increased tear strength, both when wet and when dry.

The invention is described further hereinafter, by way of example only, with reference to the accompanying drawings, in which:

Fig.1 shows a portion of a block of cellular plastics material prior to forming in accordance with one aspect of the present invention;

Fig.2 is a diagrammatic side elevation of one embodiment of a substrate in accordance with this invention, produced from the cellular material component shown in Fig.1;

Fig.3 is a plan view of the substrate of Fig.2;

Fig.4 is a diagrammatic plan view showing a pair of heated platens or jaws for use in forming the substrate of Figs. 2 and 3;

Fig.5 is a further plan view of the two platens of Fig.4, showing the platens in their "closed" position;

Fig.6 is a front elevation showing the configuration of one of the platens of Figs. 4 and 5;

Fig.7 is a bottom plan view of a further embodiment of the present invention; and

Fig.8 is a section on the line A-A in Fig.7.

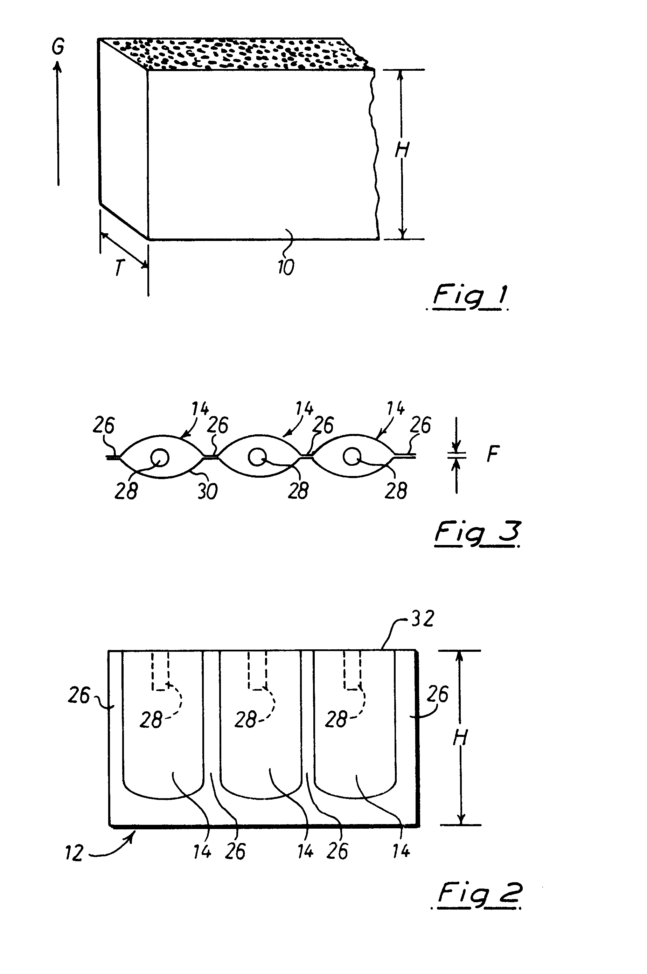

Fig.1 shows a block 10 of low density cellular foam material, such as a polyurethane foam or expanded phenolic. The block 10 has a height H, and a thickness T. Such a foam is inherently substantially hetero-cellular (i.e. open-celled). The material exhibits a "grain" parallel to its "rise", the grain direction being indicated by the arrow G in Fig.1.

The block of foam 10 shown in Fig.1 is used as a blank for the formation of the bandolier 12 of individual, low density planting (rooting or cutting) cells 14 shown in Figs. 2 and 3.

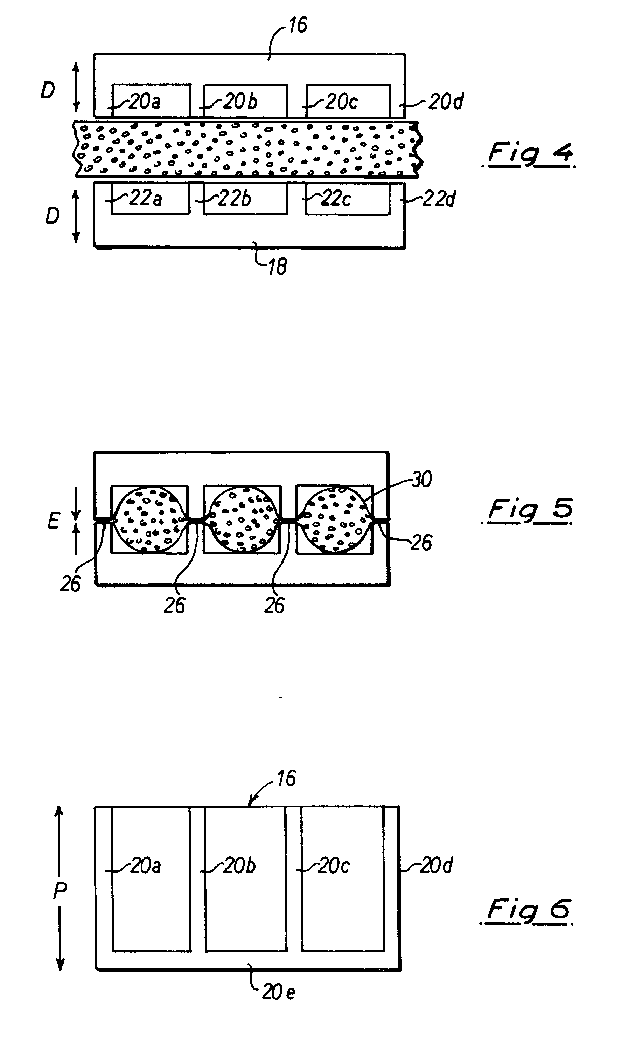

The formation of the bandolier of Figs. 2 and 3 is achieved by the use of the pair of heated platens 16, 18 shown in Figs. 4 and 5 which are selectably displaceable towards and away from one another, as indicated by the arrows D in Fig.4. Each platen 16, 18 carries a respective projecting land 20,22 having the configuration best seen in Fig.6. Thus, for example, the platen 16 has four vertical land portions 20a, 20b, 20c, 20d interconnected at their lower ends by a horizontal land portion 20e. (In practice, the number of vertical land portions is chosen to suit the length of bandolier required).

The foam blank 10 is placed between the heated platens 16,18 as shown in Fig.4, the height H of the blank being chosen to be substantially equal to the height P of the platens. The platens 16,18 are then moved towards each other until the opposing lands are separated by a small distance E. A typical value for E might in practice be a distance of the order of 0.06 cms. In this condition, the cellular material between the opposing lands is subjected to heat and pressure and the blank assumes the deformed configuration shown in Fig.5. When the platens are subsequently moved apart again, the deformation remains and one thus obtains the configuration shown in Figs. 2 and 3 having (in this case) three areas 14 of relatively low density separated, and surrounded on three sides, by membraneous areas 26 of relatively high density having a thickness F corresponding substantially to the platen spacing E of Fig.5.

A respective blind bore 28 can be formed in each low density area by any convenient means, for eventually receiving a cutting or seedling to be propagated.

By this means, there is formed a plurality of discrete, low density bandoliered cells 14 having their vertical plane parallel with the grain G of the foam. The exposed side walls 30 of the low density cells 14 have a slightly densified surface, which in turn is contiguous (apart from its upper insertion plane 32) with the highly densified membrane 26.

In use, this structure results in the encouragement of a predominantly downward root development for cuttings and seedlings positioned in the bores 28. All parts of the cells are ultimately permeable to the plants' roots. However, the combination of grain and differential densification initially encourages root growth in a vertical direction rather than laterally. This has the advantage of avoiding root damage when the individual cells are eventually sub-divided.

In one practical example, the original blank has an initial thickness of 1.5 cms. and is chosen to have a density of the order of 7.5 kgs/m$^3$. After formation using the foregoing technique, the core density of the cells 14 remains at 7.5 kgs/m$^3$. However, the surface density of the cells 14 is about 8.75 kgs/m$^3$ and the density of the interconnecting membrane 26 which has a thickness of the order of 0.06 cms. is of the order of 125 kg/m$^3$.

A comparative capillarity test on the latter example revealed the following:

A strip of polyurethane foam 1.5 cms. thick by 4.0 cms. high and 25 cms. long of a density of 7.5 kgs/m$^3$ (representing the base material of the bandolier) was placed in a tray containing pigmented water at a height of 4mm. Placed in the same tray was a 10 cell bandolier unit made in accordance with the aforegoing technique. Both samples were completely devoid of moisture prior to immersion. Examination after 24 hours revealed the following:

In the case of the undensified foam strip, the average height of the water migration was 16 mm, the foam in this area being waterlogged.

In the case of the bandolier made in accordance with the present invention, water had reached the top of the bandolier. However, it was observed that the water had been absorbed preferentially by the densified regions, i.e. in the interconnecting membranes 26.

Thus, the use of the densified regions provides a means of achieving adequate water supply to cuttings or seedlings in the cells 14 whilst avoiding the problem of waterlogging. Since the roots do not have to sit in water as can happen with known substrates, there is increased aeration of the roots. Furthermore, due to increased capillarity of the densified areas, the water depth into which the substrate must be submerged is very much less than hitherto. Hence the quantity of water necessary can be correspondingly reduced, with a consequential saving in energy requirements.

It should be emphasised that the invention is by no means limited to the particular configuration of cutting or seedling-receiving cell 14 shown in the drawings, and any convenient configuration can be chosen to suit the circumstances. Such cells need not be formed as part of a bandolier. They may, for example, be singly-formed cells or they may be formed from a block divided into parallel rows of cells by two sets of mutually orthogonal dividing grooves or channels. Densified regions are formed in such a block, for example at the base of the grooves or channels, for achieving enhanced capillarity in accordance with the invention.

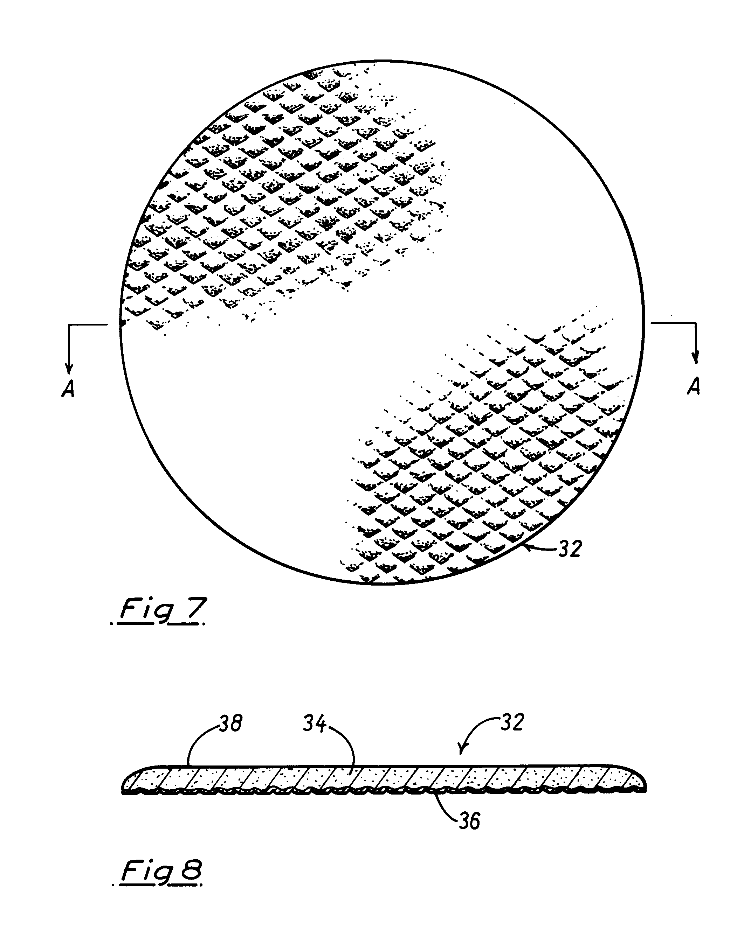

The invention also has applicability to capillary matting for use in providing water and/or nutrients to plant containers or trays placed thereabove. Figs. 7 and 8 show one example of such an embodiment in the form of a generally planar disc 32, a major part 34 of which is formed of a low density heterocellular foam material, such as a polyurethane foam or an expanded phenolic. However, the underside of the low density part 34 carries a portion 36 of relatively high density formed, for example, by subjecting one surface of the low density material to selective heat treatment in a heated press. Preferably, at least the underside surface of the higher density part 36 is formed with a pattern of ridges and recesses (for example as shown in Fig.7) so that when the disc 32 is placed on a flat surface in the orientation shown in Fig.8, contact is only made with said flat surface by said ridges. The upperside surface 38 of the disc 32 can be plain or profiled/patterned as required.

Although shown as a disc, the matting could have any desired peripheral configuration dependent on the size and shape or the plant trays or containers with which it is to be used.

## Claims

1. A synthetic substrate for use in the rooting of cuttings and the raising of seedlings, characterised by an expanded, semi-rigid, substantially hetero-cellular plastics material having at least one region (14) of relatively low density which exhibits relatively low capillarity and which is arranged, in use, to receive a cutting or seedling to be propagated and at least one region (26) of relatively high density which exhibits higher capillarity than said low density region.

2. A synthetic substrate as claimed in claim 1, having a plurality of said regions (14) of relatively low density which are adapted to receive, in use, cuttings or seedlings to be propagated, the regions (14) of low density being separated by the regions (26) of relatively high density which exhibit higher capillarity than said regions (14) of low density.

3. A synthetic substrate as claimed in claim 2, wherein the substrate is in the form of a bandolier having a plurality of said low density regions (14), separated in the longitudinal direction of the bandolier by narrow strip regions (26) of said higher density.

4. A synthetic substrate as claimed in claim 3, wherein the low density regions (14) are of generally oval transverse section.

5. A synthetic substrate as claimed in claim 3 or 4, wherein the low density regions contain one or more blind bores (28) for receiving cuttings or seedlings.

6. A synthetic substrate as claimed in any of claims 1 to 5, wherein the cellular material is a polyurethane foam or an expanded phenolic.

7. A synthetic substrate as claimed in claim 1, comprising a first, elongate portion of said relatively low density which exhibits relatively low capillarity, said first portion containing at least one blind bore which opens in an end surface of said first portion for receiving a cutting or seedling to be propagated, and a second portion of said relatively high density which exhibits higher capillarity than said first portion and which surrounds the whole of said first portion, except for said end surface containing the blind bore.

8. The use of a synthetic substrate as capillary matting for transmitting water and/or nutrients to plants, wherein the substrate comprises at least one said region of relatively low density which exhibits relatively low capillarity and on which, in use, one or more plant containers or trays can be placed, and at least one said region of relatively high density disposed below said region of relatively low density and exhibiting higher capillarity than said low density region.

9. The use of a synthetic substrate as claimed in claim 8, wherein the substrate is in the form of a generally planar lamina whose two opposite surfaces are defined by said regions of relatively high and relatively low density, respectively.

**Revendications**

1. Substrat synthétique utilisé pour l'enracinement de boutures et la culture de jeunes plants, caractérisé par une matière plastique expansée, semi-rigide, essentiellement hétérocellulaire comportant ou moins une région (14) de densité relativement faible qui présente une capillarité relativement faible et qui est constituée pour recevoir, en service, une bouture ou un plant à faire pousser et au moins une région (26) de densité relativement élevée qui présente une capillarité plus élevée que ladite région à densité faible.

2. Substrat synthétique selon la revendication 1, comportant une pluralité desdites régions (14) de densité relativement faible, ces régions étant adaptées à recevoir, en service, des boutures ou des plans à faire pousser, les régions (14) de faible densité étant séparées par les régions (26) de densité relativement élevée qui présentent une capillarité plus élevée que lesdites régions (14) de faible densité.

3. Substrat synthétique selon la revendication 2, dans lequel le substrat se présente sous la forme d'une chaîne comportent une pluralité desdites régions de faible densité (14) séparées dans la direction longitudinale de la chaîne par des régions en bandes étroites (26) présentant ladite densité plus élevée.

4. Substrat synthétique selon la revendication 3, dans lequel les régions de faible densité (14) ont une section transversale essentiellement ovale.

5. Substrat synthétique selon la revendication 3 ou 4, dans lequel les régions à faible densité comportent un ou plusieurs alésages borgnes (28) destinés à recevoir des boutures ou des plans.

6. Substrat synthétique selon l'une quelconque des revendications 1 à 5, dans lequel le matériau cellulaire est un polyuréthane cellulaire ou une matière phénolique expansée.

7. Substrat synthétique selon la revendication 1, comportant une première partie allongée ayant ladite densité relativement faible qui présente une capillarité relativement faible, ladite première partie comprenant au moins un alésage borgne qui débouche dans une surface d'extrémité de ladite première partie pour recevoir une bouture ou un plant à faire pousser, et une deuxième partie ayant ladite densité relativement élevée, qui présente une capillarité plus élevée que ladite première partie et qui entoure la totalité de ladite première partie, sauf en ce qui concerne ladite surface d'extrémité comprenant l'alésage borgne.

8. Utilisation d'un substrat synthétique en tant que tapis capillaire pour transmettre de l'eau et/ou des substances nutritives à des plantes, dans laquelle le substrat comporte au moins une dite région de densité relativement faible qui présente une capillarité relativement faible et sur laquelle, en service, on peut placer un ou plusieurs récipients ou plateaux à fleurs, et au moins une dite région de densité relativement élevée disposée sous ladite région de densité relativement faible et présentant une capillarité plus élevée que ladite région à densité faible.

**9.** Utilisation d'un substrat synthétique selon la revendication 8, dans laquelle le substrat se présente sous forme d'une feuille essentiellement plane dont les deux surfaces opposées sont respectivement définies par lesdites régions de densité relativement élevée et de densité relativement faible.

**Patentansprüche**

**1.** Synthetisches Substrat zur Verwendung beim Einpflanzen von Ablegern und beim Ziehen von Sämlingen, gekennzeichnet durch einen geschäumten, halbstarren, im wesentlichen heterozellularen Kunststoff mit wenigstens einem Bereich (14), der eine relativ niedrige Dichte hat, eine relativ niedrige Kapillarität aufweist und bei der Verwendung so angeordnet ist, daß er einen fortzupflanzenden Ableger oder Sämling aufnimmt, und mit wenigstens einem Bereich (26), der eine relativ hohe Dichte hat und eine höhere Kapillarität als der Bereich mit niedriger Dichte aufweist.

**2.** Synthetisches Substrat nach Anspruch 1, welches eine Vielzahl der genannten Bereiche (14) mit relativ niedriger Dichte aufweist, die dafür geeignet sind, bei der Verwendung fortzupflanzende Ableger oder Sämlinge aufzunehmen, wobei die Bereiche (14) mit niedriger Dichte durch die Bereiche (26) mit relativ hoher Dichte getrennt sind, welche eine höhere Kapillarität als die Bereiche (14) mit niedriger Dichte haben.

**3.** Synthetisches Substrat nach Anspruch 2, bei welchem das Substrat die Form eines Bandes hat, welches eine Vielzahl von Bereichen (14) niedriger Dichte aufweist, die in Längsrichtung des Bandes durch schmale Streifenbereiche (26) mit höherer Dichte getrennt sind.

**4.** Synthetisches Substrat nach Anspruch 3, bei welchem die Bereiche (14) mit niedriger Dichte insgesamt einen ovalen Querschnitt haben.

**5.** Synthetisches Substrat nach Anspruch 3 oder 4, bei welchem die Bereiche mit niedriger Dichte ein oder mehrere Sacklöcher (28) für die Aufnahme von Ablegern oder Sämlingen aufweisen.

**6.** Synthetisches Substrat nach einem der Ansprüche 1 bis 5, bei welchem das zellulare Material ein Polyurethanschaum oder geschäumtes Phenolharz ist.

**7.** Synthetisches Substrat nach Anspruch 1, welches einen ersten, langgestreckten Abschnitt, der eine relativ niedrige Dichte und eine relativ niedrige Kapillarität hat und wenigstens ein Sackloch enthält, das in eine Stirnfläche des ersten Abschnitts für die Aufnahme eines fortzupflanzenden Ablegers oder Sämlings mündet, und einen zweiten Abschnitt aufweist, der eine relativ hohe Dichte und eine höhere Kapillarität als der erste Abschnitt hat und den gesamten ersten Abschnitt mit Ausnahme der das Sackloch enthaltenden Stirnfläche umgibt.

**8.** Verwendung eines synthetischen Substrats als Kapillarmatte zur Übertragung von Wasser und/oder Nährstoffen auf Pflanzen, wobei das Substrat wenigstens einen Bereich hat, der eine relativ niedrige Dichte und eine relativ niedrige Kapillarität aufweist und auf welchem bei der Verwendung ein oder mehrere Pflanzenbehälter oder Tröge plaziert werden können, und wenigstens ein Bereich mit relativ hoher Dichte unter dem Bereich mit relativ niedriger Dichte angeordnet ist und eine höhere Kapillarität als der Bereich mit niedriger Dichte aufweist.

**9.** Verwendung eines synthetischen Substrats nach Anspruch 8, wobei das Substrat die Form einer insgesamt ebenflächigen Platte hat, deren beide gegenüberliegende Oberflächen von den Bereichen relativ hoher bzw. relativ niedriger Dichte gebildet werden.

_Fig 1_

_Fig 3_

_Fig 2_

16

D

20a    20b    20c    20d

_Fig 4_.

22a    22b    22c    22d

18

D

30

E

_Fig 5_.

26

26    26    26

16

20a    20b    20c    20d

P

_Fig 6_.

20 e

EP 0 252 191 B1

_Fig 7_

_Fig 8_

9